(19) Europäisches Patentamt

European Patent Office

Office européen des brevets

(11) **EP 2 000 154 A2**

(12) **EUROPEAN PATENT APPLICATION**
published in accordance with Art. 153(4) EPC

(43) Date of publication:
**10.12.2008 Bulletin 2008/50**

(21) Application number: **07736940.3**

(22) Date of filing: **23.03.2007**

(51) Int Cl.:
**A61K 51/00** (2006.01)   **C07F 13/00** (2006.01)

(86) International application number:
**PCT/JP2007/000283**

(87) International publication number:
**WO 2007/111020 (04.10.2007 Gazette 2007/40)**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **24.03.2006 JP 2006083664**

(71) Applicant: **Fujifilm RI Pharma Co., Ltd.**
**Chuo-ku**
**Tokyo 1040031 (JP)**

(72) Inventors:
• **KITAJIMA, Akihito**
  **Sammu-shi, Chiba 289-1592 (JP)**
• **SAWANO, Kaita**
  **Sammu-shi, Chiba 289-1592 (JP)**
• **MATSUSHIMA, Satoshi**
  **Sammu-shi, Chiba 289-1592 (JP)**

(74) Representative: **Blodig, Wolfgang et al**
**Wächtershäuser & Hartz**
**Patentanwälte**
**Weinstrasse 8**
**80333 München (DE)**

(54) **COMPOSITION FOR PREPARATION OF RADIOACTIVE PHARMACEUTICAL FOR DIAGNOSIS OF REGIONAL CEREBRAL BLOOD FLOW**

(57)    An object of the present invention is to provide a composition for effectively labeling ECD with 99mTc within a short period of time.

The composition for producing [N,N'-ethylenedi-L-cysteinate(3-)]oxotechnetium(99mTc) diethyl ester is **characterized by** containing N,N'-(1,2-ethylene)bis-L-cysteine diethyl ester or a salt thereof, a reducing agent, and an acidic substance or a salt thereof.

**Description**

Technical Field

The present invention relates to a composition for producing a technetium-labeled radiopharmaceutical, which is employed in diagnosis of cerebral blood flow through cerebral functional imaging.

Background Art

The brain, which is a very important life-supporting organ, functions by taking oxygen from a sufficient amount of blood constantly supplied to the brain. However, when blood flow is reduced or stopped by some disorder occurring in a cerebral blood vessel, cerebral functions are impaired, leading to necrosis of brain cells. Thus, in order to monitor blood flow, some blood flow diagnosing methods have been developed.

Hitherto, there have been studied cerebral blood flow-imaging agents. Among them, diaminedithiol (hereinafter abbreviated as DADT) compounds are known to readily form a chelate with radioactive technetium. By virtue of their structural features, these compounds have been considered effective brain-imaging agents. However, since such compounds, while uptaken by the brain at high efficiency, are generally discharged rapidly from the brain, use of these compounds as imaging agents has not been realized.

In order to solve the problem, Du Pont U.S. has focused on the retention mechanism of an ester group in the cerebral parenchyma and studied ester-group-introduced DADT compounds. Through an imaging test of a variety of ester-group-introduced DADT compounds in monkeys, Cheesman et *al.* have found that [N,N'-ethylenedi-L-cysteinate (3-)]oxotechnetium(99mTc) diethyl ester (hereinafter referred to as 99mTc-ECD) and similar compounds are uptaken by the brain at high efficiency and retained in the brain for a long time, which are suitable cerebral blood flow imaging compounds (Patent Document 1). The retention mechanism is considered to be as follows. Specifically, an ester-group-introduced DADT compound is uptaken by the cerebral parenchyma through the blood-brain barrier and enzymatically decomposed in the brain, to thereby form a polar compound as a metabolite. Since the polar compound has no permeability to the blood-brain barrier, the compound can be retained in the cerebral parenchyma.

Walovitch et al. have confirmed, through various experiments by use of brain homogenates, that an ester group in 99mTc-ECD is hydrolyzed in the brain tissue to thereby rapidly form a polar compound as a metabolite having no permeability to the blood-brain barrier, and that the polar compound is accumulated in the cerebral parenchyma in proportion to the cerebral blood flow, resulting in long-time retention in brain cells. In addition, since an ester group in 99mTc-ECD has low affinity for hemocytes and soft tissue and attains rapid clearance from blood and organs other than the brain, an image with low background can be obtained. Therefore, 99mTc-ECD is a useful radiopharmaceutical for diagnosis of cerebral blood flow and, in fact, is now widely used as such.

Pharmaceutical products containing 99mTc-ECD which are currently available on the market are each composed of two vials (vial A and vial B), which are intended to be reconstituted into an injection upon use. Vial A is prepared by lyophilizing a solution of composition A containing N,N'-(1,2-ethylene)bis-L-cysteine diethyl ester (hereinafter referred to as ECD) dihydrochloride, stannous chloride, sodium edetate, and D-mannitol, whereas vial B is a solution of composition B containing sodium dihydrogenphosphate and disodium hydrogenphosphate. Before use, 99mTc-ECD is prepared by adding 3 mL or less of 99mTc sodium pertechnetate to vial B; preparing vial A into a solution of 3 mL; adding an aliquot (1 mL) of the solution in vial A to vial B; sufficiently stirring the vial; and allowing the mixture to stand at room temperature for 30 minutes.

Patent Document 1: JP-B-1995-64802

Disclosure of the Invention

Problems to be Solved by the Invention

However, such currently available 99mTc-ECD pharmaceutical products have drawbacks. Specifically, technicians (generally doctors and radiological technicians) who attended to reconstitution of the products must perform time-consuming work including measurement of reaction time. Also, in situations where an emergency test is needed, use of a reconstitution-type injection is limited. Thus, to deal with an emergency test which must be performed immediately, in actual medical settings, there is keen demand for a pharmaceutical composition which can considerably shorten the time for preparing 99mTc-ECD.

An object of the present invention is to provide a pharmaceutical composition for effectively labeling ECD with 99mTc within a short period of time.

Means for Solving the Problems

The present inventors have carried out extensive studies for the purpose of shortening time of labeling reaction of ECD with 99mTc, and have found that, when 99mTc-labeling reaction is performed in the presence of ECD, a reducing agent, and an acidic substance or a salt thereof, the reaction time is considerably shortened, whereby a radiopharmaceutical for diagnosis of local cerebral blood flow, the pharmaceutical being adapted to an emergency test, can be produced. The present invention has been accomplished on the basis of this finding.

Accordingly, the present invention provides a composition for producing [N,N'-ethylenedi-L-cysteinate(3-)]oxotechnetium(99mTc) diethyl ester comprising N,N'-(1,2-ethylene)bis-L-cysteine diethyl ester or a salt thereof, a reducing agent, and an acidic substance or a salt thereof.

The present invention also provides a method for producing [N,N'-ethylenedi-L-cysteinate(3-)]oxotechnetium(99mTc) diethyl ester, which comprises reacting N,N'-(1,2-ethylene)bis-L-cysteine diethyl ester or a salt thereof with 99mTc pertechnetic acid or a salt thereof, in the presence of a reducing agent and an acidic substance or a salt thereof.

The present invention also provides an injection containing [N,N'-ethylenedi-L-cysteinate(3-)]oxotechnetium(99mTc) diethyl ester produced through the aforementioned production method.

Effects of the Invention

Through use of the composition of the present invention for producing 99mTc-ECD, high purity 99mTc-ECD with extremely low impurity level can be produced within a very short time.

According to the present invention, an emergency test for checking cerebrovascular disorders and cerebral function disorders and the like can be performed through cerebral blood flow imaging.

Brief Description of the Drawings

[Fig. 1]
The figure showing the relationship between the time after the start of labeling reaction and radiochemical purity of 99mTc-ECD, in the cases where vial B contains composition B and where vial B contains ascorbic acid solution instead of composition B.

[Fig. 2]
The figure showing the relationship between the ascorbic acid solution concentration and radiochemical purity of 99mTc-ECD at 0.5, 1, and 5 minutes after the start of labeling reaction, where composition B contained in vial B is changed to ascorbic acid solution.

Best Modes for Carrying Out the Invention

In the method for producing 99mTc-ECD according to the present invention, ECD or a salt thereof is reacted with 99mTc pertechnetic acid or a salt thereof in the presence of a reducing agent and an acidic substance or a salt thereof. Examples of the ECD salt include acid-added salts of ECD; e.g., an ECD hydrochloride, an ECD sulfate, and an ECD nitrate. Of these, ECD hydrochloride is preferred, with ECD dihydrochloride being particularly preferred.

Examples of the reducing agent include alkali metal dithionites, stannous salts, and sodium borohydride. Of these, stannous salts are preferred, with stannous nitrate, stannous tartrate, and stannous chloride being more preferred. Particularly preferred is stannous chloride.

The amount of the reducing agent employed in the composition, which varies depending on the amount of 99mTc pertechnetic acid or a salt thereof, is preferably 0.01 to 0.10 mg with respect to 0.3 mg of ECD or a salt thereof, more preferably 0.01 to 0.05 mg, particularly preferably 0.024 mg.

A characteristic feature of the present invention resides in use of an acidic substance or a salt thereof. The reason for considerably shortening the 99mTc-labeling reaction time by an acidic substance or a salt thereof has not been clearly elucidated. However, one possible reason is that the acidic substance or the salt thereof accelerates the reduction of the labeling agent by the reducing agent. Examples of the acidic substance or the salt thereof include ascorbic acid and a salt thereof, citric acid and a salt thereof, sulfurous acid and a salt thereof, gentisic acid and a salt thereof, a thiosulfate salt, a pyrosulfite salt, and a hydrogen sulfite salt. These species may be used singly or in combination of two or more species. Among these acidic substance salts, alkali metal salts such as sodium salts and potassium salts are preferred. Of these, ascorbic acid, sodium ascorbate, citric acid, sodium citrate, sodium sulfite, gentisic acid, sodium gentisate, sodium thiosulfate, sodium pyrosulfite, and sodium hydrogen sulfite are more preferred.

The amount of the acidic substance or the salt thereof employed in the composition is preferably 1 to 600 mg with respect to 0.3 mg of ECD or a salt thereof, particularly preferably 5 to 150 mg. Specifically, ascorbic acid or a salt thereof is preferably used in an amount of 10 to 600 mg, particularly preferably 50 to 200 mg, with respect to 0.3 mg of ECD or a salt thereof. Citric acid or a salt thereof is preferably used in an amount of 1 to 100 mg, particularly preferably 10 to 100 mg, with respect to 0.3 mg of ECD or a salt thereof. Sulfurous acid or a salt thereof is preferably used in an amount of 1 to 100 mg, particularly preferably 20 to 50 mg, with respect to 0.3 mg of ECD or a salt thereof. Gentisic acid or a salt thereof is preferably used in an amount of 1 to 20 mg, particularly preferably 7.5 mg, with respect to 0.3 mg of ECD or a salt thereof. Thiosulfate salt is preferably used in an amount of 1 to 100 mg, particularly preferably 5 to 20 mg, with respect to 0.3 mg of ECD or a salt thereof. Pyrosulfite salt is preferably used in an amount of 1 to 100 mg, particularly preferably 5 to 20 mg, with respect to 0.3 mg of ECD or a salt thereof. Hydrogen sulfite salt is preferably used in an amount of 1 to 100 mg, particularly preferably 10 to 50 mg, with respect to 0.3 mg of ECD or a salt thereof. Preferably, thiosulfate salt is used in combination with benzyl alcohol.

99mTc pertechnetic acid or a salt thereof which is the labeling agent is preferably an alkali metal salt such as 99mTc sodium pertechnetate. The labeling agent is preferably used in an amount, with respect to 0.3 mg of ECD or a salt thereof, of 37 to 7,400 MBq at labeling, particularly preferably 600 MBq. The labeling agent is preferably prepared before labeling reaction through a known method; e.g., by means of a sodium pertechnetate(99mTc) injection generator.

The labeling reaction is preferably performed in a solution having a pH of 6 to 9, particularly preferably in a buffer solution having a pH of 6 to 9, from the viewpoints of reaction efficiency, use as an injection after labeling, and labeling purity. No particular limitation is imposed on the buffer solution having a pH of 6 to 9, so long as the buffer solution is an aqueous solution containing a buffer which can control the pH to 6 to 9. Examples of the buffer include phosphate buffers, citrate buffers, and tartrate buffers. Of these, a buffer having a pH of 6 to 8 is preferred from the viewpoints of reaction efficiency and use as an injection after labeling, and a phosphate buffer is more preferred. Particularly preferred is a mixture of sodium dihydrogenphosphate and disodium hydrogenphosphate.

In the labeling reaction solution, a chelating agent such as sodium edetate, sodium diethylenetriaminepentaacetate, or cyclohexanediaminetetraacetic acid; a stabilizer such as D-mannitol, lactose, or glucose; a preservative such as benzyl alcohol; a tonicity agent such as sodium chloride; or other additives may be present.

The labeling reaction is preferably performed at an ECD (or a salt thereof) concentration of 0.03 to 0.3 mg/mL, particularly preferably 0.06 to 0.1 mg/mL. The amounts of components other than ECD or a salt thereof, which also involved in the reaction, depend on the ECD concentration and are selected from the aforementioned ranges. The labeling reaction is preferably performed at 4 to 70°C, particularly at 10 to 30°C. The reaction time required is within five minutes, preferably within one minute. Thus, according to the invention, the required reaction time is remarkably shortened, as compared with conventional methods.

According to the aforementioned labeling reaction, a 99mTc-ECD-containing injection can be produced within a very short period of time in a simple manner.

As described above, generally, the labeling agent is prepared before labeling reaction. Therefore, in the present invention, a composition containing ECD or a salt thereof, a reducing agent, and an acidic substance or a salt thereof is preferably prepared in advance as a composition for producing 99mTc-ECD. In order to adjust the pH of the composition to 6 to 9, a buffer may be incorporated in advance to the composition.

When a buffer is employed, at least ECD or a salt thereof and the buffer are preferably placed in separate containers, or in a single container such that the two components are not in contact with each other, since the buffer reacts with ECD or a salt thereof.

So long as ECD or a salt thereof and the buffer are placed in separate containers, other components may be placed in any container. Thus, examples of the form of the composition of the present invention for producing 99mTc-ECD include (1) a combination of composition A containing ECD or a salt thereof and composition B containing a reducing agent, an acidic substance or a salt thereof, and a buffer; (2) a combination of composition A containing ECD or a salt thereof and a reducing agent and composition B containing an acidic substance or a salt thereof and a buffer; and (3) a combination of composition A containing ECD or a salt thereof, a reducing agent, and an acidic substance or a salt thereof and composition B containing a buffer. Of these, when a buffer is employed, the combination (2) is particularly preferred. Into these compositions, a chelating agent such as sodium edetate, sodium diethylenetriaminepentaacetate, or cyclohexanediaminetetraacetic acid; a stabilizer such as D-mannitol, lactose, or glucose; a preservative such as benzyl alcohol; a tonicity agent such as sodium chloride; or other additives may be incorporated. When no buffer is employed, the combinations (1), (2), and (3) preferably contain no buffer.

For producing an 99mTc-ECD injection by use of any of the compositions, a composition containing ECD or a salt thereof is dissolved in physiological saline or a similar medium, and a labeling agent is added to the solution, followed by adding other components. The thus-obtained solution containing 99mTc-ECD can be employed as is as a radiopharmaceutical for diagnosis of local cerebral blood flow.

According to the present invention, 99mTc-ECD having a radiochemical purity of 95% or higher can be produced through labeling reaction within five minutes, furthermore within one minute. In addition, since reaction efficiency is very high, the volume of the aforementioned composition A can be reduced to 1/2 to 1/10 compared with a conventional composition A.

Examples

The present invention will next be described in more detail by way of examples, which should not be construed as limiting the invention thereto.

Referential Example 1

Conventional 99mTc-ECD was prepared by use of Neurolite™ Daiichi (product of Daiichi Radioisotope Laboratories, Ltd.). Neurolite™ Daiichi is in the form of injection reconstituted upon use, the injection including two vials (vial A and vial B). Vial A is prepared by lyophilizing a composition containing ECD dihydrochloride, stannous chloride, sodium edetate, and D-mannitol, whereas vial B is a solution composed of sodium dihydrogenphosphate and disodium hydrogenphosphate. Labeling was performed in the following manner. By use of Ultra-Techne Kow™ (product of Daiichi Radioisotope Laboratories, Ltd.), 3 mL or less of a pertechnetic acid salt(99mTc) solution having a radioactivity of 400 to 800 MBq was added to vial B. Then, physiological saline (3 mL) was added to vial A, and the contents were dissolved with shaking. An aliquot (1 mL) of vial A solution was immediately added to vial B, and the mixture was allowed to stand at room temperature.

Referential Example 2

Method of analyzing 99mTc-ECD

The radiochemical purity of 99mTc-ECD was determined through thin-layer chromatography. When a thin-layer plate (product of Whatman) and a developer (acetonitrile : ammonium acetate = 60 : 40) were employed, 99mTc-ECD which had been prepared through the method of Referential Example 1 was developed to an Rf value of 0.30 to 0.55. A non-bonding 99mTc sodium pertechnetate present with 99mTc-ECD was developed to an Rf of 0.8 to 1.0. Thus, the radiochemical purity of 99mTc-ECD can be calculated by the following formula:

$$\text{Radiochemical purity (\%) of 99mTc-ECD} = (A_1/A_2) \times 100,$$

wherein $A_1$ represents peak radioactivity (Rf: 0.30 to 0.55), and $A_2$ represents a total radioactivity on the thin-layer plate.

Example 1

Evaluation 1 of ascorbic acid solution

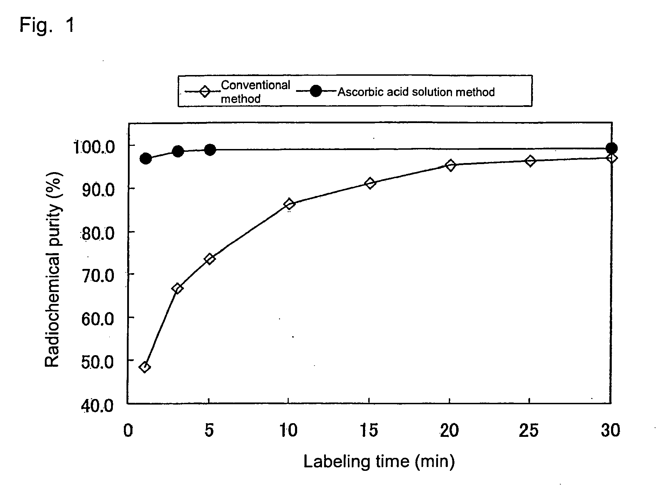

The content of vial B, which is a composition for producing Neurolite™ Daiichi, was changed to an ascorbic acid solution (1 mL) (pH: 8.0). Then, 99mTc-ECD was prepared through the procedure of Referential Example 1. Time after the start of labeling reaction and radiochemical purity were determined through the method described in Referential Example 2, and these values were compared with those obtained through the aforementioned conventional method. In Example 1, the concentration of the ascorbic acid solution was adjusted to 100 mg/mL, and the radioactivity and the volume of the solution were adjusted to 600 MBq and 4 mL. The conventional method and the ascorbic acid method, in which the content of conventional vial B had been changed to ascorbic acid solution, in preparation of 99mTc-ECD, were compared with each other in terms of time after the start of labeling reaction and radiochemical purity. The results are shown in Fig. 1.
As is clear from Fig. 1, when the conventional vial B was changed to the ascorbic acid solution, high radiochemical purity was attained immediately after the start of labeling, as compared with the conventional method.

Example 2

Evaluation 2 of ascorbic acid method

The effect of ascorbic acid solution on accelerating labeling reaction as observed in Example 1 was further investigated. Specifically, effects of variations in pH and concentration of the ascorbic acid solution on time after the start of labeling reaction and radiochemical purity were investigated.

The pH of ascorbic acid solution was varied among 6.0, 6.5, 7.0, 7.5, 8.0, 8.5, and 9.0, and the concentration (mg/mL) of the ascorbic acid solution was adjusted to 100 and 150. For each combination of pH and concentration, radiochemical purity was determined at 0.5 min, 1 min, 3 min, 5 min, and 30 min after the start of labeling, through the method described in Referential Example 2. In Example 2, the radioactivity and the volume of each solution in the labeling reaction were adjusted to 600 MBq and 4 mL.

Table 1 shows the results. As is clear from Table 1, ascorbic acid solution effectively shortened the time required for labeling over a wide pH range.

[Table 1]

| Labeling reaction time (min) | Ascorbic acid solution concentration (mg/mL) | pH 6.0 | pH 6.5 | pH 7.0 | pH 7.5 | pH 8.0 | pH 8.5 | pH 9.0 |
|---|---|---|---|---|---|---|---|---|
| 0.5 | 100 | 86.0 | 86.8 | 94.9 | 96.0 | 93.8 | 93.7 | 92.2 |
| 0.5 | 150 | 88.8 | 90.7 | 95.0 | 95.9 | 97.2 | 96.9 | 94.3 |
| 1 | 100 | 88.6 | 87.4 | 95.7 | 96.7 | 96.8 | 96.3 | 96.3 |
| 1 | 150 | 89.7 | 90.4 | 98.2 | 97.7 | 98.0 | 98.0 | 96.5 |
| 3 | 100 | 90.0 | 90.7 | 98.5 | 98.5 | 98.3 | 98.1 | 97.4 |
| 3 | 150 | 94.1 | 93.9 | 98.5 | 98.6 | 98.6 | 98.5 | 97.3 |
| 5 | 100 | 93.5 | 93.2 | 98.7 | 98.5 | 98.7 | 98.4 | 97.5 |
| 5 | 150 | 95.9 | 95.6 | 98.5 | 98.7 | 98.7 | 98.6 | 96.8 |
| 30 | 100 | 98.4 | 98.0 | 98.6 | 98.9 | 98.9 | 98.6 | 98.0 |
| 30 | 150 | 98.5 | 98.2 | 98.6 | 98.8 | 99.1 | 98.8 | 97.7 |

Labeling reaction time and radiochemical purity (%) of 99mTc-ECD, with changes in pH and concentration of ascorbic acid solution

Example 3

Evaluation 3 of ascorbic acid method

Ascorbic acid solution was added to the content of vial B, which is a composition for producing Neurolite™ Daiichi of Referential Example 1, and time after the start of labeling reaction and radiochemical purity were investigated. To vial B of Neurolite™ Daiichi, each (1 mL) of the ascorbic acid solutions having a pH of 6.0, 6.5, 7.0, 7.5, 8.0, 8.5, and 9.0, respectively was added.

The concentration (mg/mL) of the ascorbic acid solution was adjusted to 50, 100, and 150. For each combination of pH and concentration, radiochemical purity was determined at 0.5 min, 1 min, 3 min, and 30 min after the start of labeling, through the method described in Referential Example 2.

In Example 3, the radioactivity and the volume of each solution in the labeling reaction were adjusted to 600 MBq and 4 mL. Table 2 shows the results. As is clear from Table 2, ascorbic acid solution effectively shortened the time required for labeling over a wide pH range.

[Table 2]

| Labeling reaction time and radiochemical purity (%) of 99mTc-ECD, with changes in pH and concentration of ascorbic acid solution which was added to vial B of Neurolite™ Daiichi | | | | | | | | |
|---|---|---|---|---|---|---|---|---|
| Labeling reaction time (min) | Ascorbic acid solution concentration (mg/mL) | pH 6.0 | pH 6.5 | pH 7.0 | pH 7.5 | pH 8.0 | pH 8.5 | pH 9.0 |
| 0.5 | 50 | 93.6 | 91.8 | 93.1 | 92.2 | 93.7 | 91.9 | 87.3 |
| | 100 | 94.9 | 93.9 | 96.1 | 96.0 | 95.8 | 93.0 | 91.3 |
| | 150 | 94.9 | 94.1 | 97.0 | 96.4 | 97.4 | 94.7 | 94.3 |
| 1 | 50 | 94.5 | 93.4 | 94.9 | 96.2 | 95.5 | 94.7 | 93.0 |
| | 100 | 95.0 | 94.9 | 96.9 | 97.2 | 97.2 | 96.4 | 95.5 |
| | 150 | 95.0 | 95.9 | 97.6 | 97.7 | 97.8 | 97.3 | 97.0 |
| 3 | 50 | 97.6 | 97.4 | 96.6 | 96.8 | 97.0 | 96.7 | 96.3 |
| | 100 | 98.7 | 98.1 | 97.8 | 98.0 | 98.0 | 97.8 | 97.1 |
| | 150 | 98.6 | 98.5 | 98.1 | 98.3 | 98.4 | 97.9 | 97.4 |
| 30 | 50 | 98.8 | 98.6 | 98.3 | 98.4 | 99.1 | 98.6 | 98.0 |
| | 100 | 98.8 | 98.6 | 98.4 | 98.6 | 99.0 | 98.6 | 97.7 |
| | 150 | 98.9 | 98.6 | 98.4 | 98.6 | 99.0 | 98.6 | 97.8 |

Example 4

Evaluation 4 of ascorbic acid method

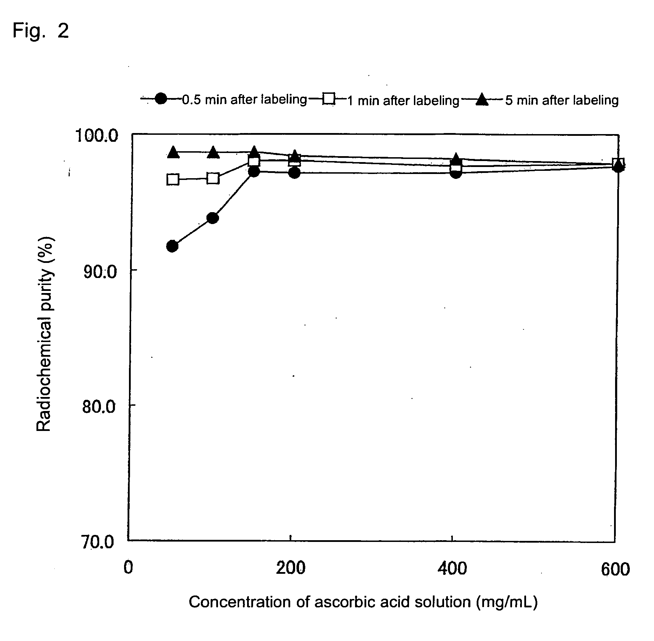

The effect of ascorbic acid solution on accelerating labeling reaction as observed in Example 1 was further investigated. Specifically, an effect of variation in concentration of the ascorbic acid solution on time after the start of labeling reaction and radiochemical purity were investigated.
The content of vial B, which is a composition for producing Neurolite™ Daiichi, was changed to an ascorbic acid solution (1 mL) (pH: 8.0) having a concentration (mg/mL) of 50, 100, 150, 200, 400, or 600. In Example 4, the radioactivity and the volume of each solution in the labeling reaction were adjusted to 600 MBq and 4 mL. Radiochemical purity was determined at 0.5 min, 1 min, and 5 min after the start of labeling, through the method described in Referential Example 2. Fig. 2 shows the results. As is clear from Fig. 2, in all measurements, a high radiochemical purity of 90% or more was attained after the start of labeling.

Example 5

Evaluation when sodium thiosulfate was employed

Sodium thiosulfate and benzyl alcohol were added to the content of vial B, which is a composition for producing Neurolite™ Daiichi of Referential Example 1, and time after the start of labeling reaction and radiochemical purity were investigated.
The content (Eq) of vial B of known Neurolite™ Daiichi was varied to 1/10, 1/4, 1/2, and 1, and sodium thiosulfate (10 mg) and benzyl alcohol (27.0 µL) were added to each sample. Water was added to each mixture, to thereby adjust the volume thereof to 1 mL. Each of the thus-obtained solutions was analyzed in terms of the time after the start of labeling for preparation of 99mTc-ECD and radiochemical purity, through conventional methods, and the relationship therebetween was investigated. In each sample, radiochemical purity was determined at 0.5 min, 1 min, 5 min, and 30 min after the start of labeling, through the method described in Referential Example 2. In Example 5, the radioactivity and the volume of each solution in the labeling reaction were adjusted to 600 MBq and 4 mL. Table 3 shows the results.
As is clear from Table 3, through addition of sodium thiosulfate and benzyl alcohol, a high radiochemical purity was attained immediately after the start of labeling, and the labeling time was considerably shortened.
[Table 3]

| Effect of sodium thiosulfate on accelerating labeling reaction | | | | |
|---|---|---|---|---|
| Amount of vial B content (Eq) | Radiochemical purity (%) | | | |
| | 0.5 min | 1 min | 5 min | 30 min |
| 1/10 | 96.2 | 97.1 | 96.8 | 96.4 |
| 1/4 | 96.7 | 96.8 | 96.9 | 97.0 |
| 1/2 | 95.0 | 95.0 | 96.6 | 97.0 |
| 1 | 92.2 | 94.6 | 96.1 | 96.9 |

Example 6

Evaluation of a sulfite salt

A solution (1 mL) of a sulfite salt (sodium sulfite, sodium hydrogen sulfite, or sodium pyrosulfite) was added to the content of vial B, which is a composition for producing Neurolite™ Daiichi of Referential Example 1. Each of the thus-obtained samples was analyzed in terms of the time after the start of labeling for preparation of 99mTc-ECD and radiochemical purity, through conventional methods, and the relationship therebetween was investigated. The amount (mg/mL) of sodium sulfite added was varied to 40 and 50; that of sodium hydrogen sulfite added was varied to 20, 30, 40, and 50; and that of sodium pyrosulfite added was varied to 10 and 20. In each sample, radiochemical purity was determined at 5 min and 30 min after the start of labeling, through the method described in Referential Example 2. In Example 6, the radioactivity and the volume of each solution in the labeling reaction were adjusted to 600 MBq and 4 mL. Table 4 shows the results. As is clear from Table 4, through addition of a sulfite salt, a high radiochemical purity was attained immediately after the start of labeling, and the labeling time was considerably shortened.
[Table 4]

| Effect of sulfite salts on accelerating labeling reaction | | | |
|---|---|---|---|
| Sulfite | Amount (mg) | Radiochemical purity (%) | |
| | | 5 min | 30 min |
| Sodium sulfite | 40 | 88.0 | 96.5 |
| | 50 | 90.6 | 96.6 |
| Sodium hydrogen sulfite | 20 | 97.7 | 97.7 |
| | 30 | 96.6 | 96.6 |
| | 40 | 96.4 | 96.0 |
| | 50 | 94.9 | 94.3 |
| Sodium pyrosulfite | 10 | 96.3 | 97.7 |
| | 20 | 97.2 | 96.9 |

Example 7

Evaluation of a citrate salt

A solution (1 mL) of citric acid or sodium citrate was added to the content of vial B, which is a composition for producing Neurolite™ Daiichi of Referential Example 1. Each of the thus-obtained samples was analyzed in terms of the time after the start of labeling for preparation of 99mTc-ECD and radiochemical purity, through conventional methods, and the relationship therebetween was investigated. The amount (mg/mL) of citric acid added was varied to 20, 50, and 100; and that of sodium citrate added was varied to 10, 50, and 100. In each sample, radiochemical purity was determined after the start of labeling, through the method described in Referential Example 2. In Example 7, the radioactivity and the volume of each solution were adjusted to 600 MBq and 4 mL.

Table 5 shows the results. As is clear from Table 5, through addition of citric acid or a salt thereof, a high radiochemical purity was attained immediately after the start of labeling, and the labeling time was considerably shortened.

[Table 5]

| Effect of citrate salts on accelerating labeling reaction | | | | |
|---|---|---|---|---|
| Citrate | Amount (mg) | Radiochemical purity (%) | | |
| | | 1 min | 5 min | 30 min |
| Citric acid | 20 | 94.7 | 97.6 | 97.3 |
| | 50 | 96.0 | 97.5 | 96.4 |
| | 100 | 94.0 | 94.4 | 92.2 |
| Sodium citrate | 10 | | 90.1 | 96.9 |
| | 50 | | 90.2 | 96.8 |
| | 100 | | 90.4 | 96.1 |

**Claims**

1. A composition for producing [N,N'-ethylenedi-L-cysteinate(3-)]oxotechnetium(99mTc) diethyl ester comprising N, N'-(1,2-ethylene)bis-L-cysteine diethyl ester or a salt thereof, a reducing agent, and an acidic substance or a salt thereof.

2. A composition as described in claim 1, wherein the reducing agent is an alkali metal dithionite, a stannous salt, or sodium borohydride.

3. A composition as described in claim 1 or 2, wherein the acidic substance or the salt thereof is one or more species selected from among ascorbic acid or a salt thereof, citric acid or a salt thereof, sulfurous acid or a salt thereof, gentisic acid or a salt thereof, a thiosulfate salt, a pyrosulfite salt, and a hydrogen sulfite salt.

4. A composition as described in any one of claims 1 to 3, which further contains a buffer for adjusting the pH of the composition to 6 to 9.

5. A composition as described in claim 4, wherein at least N,N'-(1,2-ethylene)bis-L-cysteine diethyl ester or a salt thereof and the buffer are placed in separate containers, or in a single container such that the two components are not in contact with each other.

6. A method for producing [N,N'-ethylenedi-L-cysteinate(3-)]oxotechnetium(99mTc) diethyl ester, which comprises reacting N,N'-(1,2-ethylene)bis-L-cysteine diethyl ester or a salt thereof with 99mTc pertechnetic acid or a salt thereof, in the presence of a reducing agent and an acidic substance or a salt thereof.

7. A production method as described in claim 6, wherein the reaction is performed in a buffer solution having a pH of 6 to 9.

8. An injection containing [N,N'-ethylenedi-L-cysteinate(3-)]oxotechnetium(99mTc) diethyl ester produced through a production method as described in claim 6 or 7.

Fig. 1

Fig. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7064802 B